Europäisches Patentamt

European Patent Office

Office européen des brevets

(11) Publication number: **0 080 032**
A2

# (12) EUROPEAN PATENT APPLICATION

(21) Application number: 82107860.7

(22) Date of filing: 26.08.82

(51) Int. Cl.³: **A 61 K 45/02**

(30) Priority: 20.11.81 US 323575

(71) Applicant: **ENZO BIOCHEM, INC., 325 Hudson Street, New York, N.Y. 10013 (US)**

(43) Date of publication of application: 01.06.83
**Bulletin 83/22**

(72) Inventor: **Engelhardt, Dean, 173 Riverside Drive, New York, N.Y. 10032 (US)**

(84) Designated Contracting States: **AT BE CH DE FR GB IT LI LU NL SE**

(74) Representative: **Vossius Vossius Tauchner Heunemann Rauh, Siebertstrasse 4 P.O. Box 86 07 67, D-8000 München 86 (DE)**

(54) Pharmaceutical preparation for treating herpetic lesions.

(57) Described are pharmaceutical preparations which contain human interferon and a carrier, such as distilled water. The pharmaceutical preparation may also contain a stabilizer, a preservative, an antibiotic, an anti-viral agent, and an anti-inflammatory agent. The preparations are useful for local treatment of herpetic lesions, especially for treating Herpes labialis and Herpes genitalis lesions.

EP 0 080 032 A2

0080032

25. Aug. 1982

UCHNER

SIEBERTS... MÜNCHEN 80

Our Ref.: S 057 EP
1 Case: 18588-EPO

ENZO BIOCHEM, INC.
NEW YORK, N.Y., U.S.A.

### PHARMACEUTICAL PREPARATION FOR TREATING HERPETIC LESIONS

This invention relates to the treatment of infections caused by Herpes simplex virus.  More particularly, this invention relates to the treatment of Herpes labialis and Herpes genitalis in humans by topical administration of human interferon.

Infections with Herpes simplex virus are extremely common in man throughout the world.  Primary infection by the virus usually occurs early in life.  In its most common clinical form, Herpes labialis, the symptoms include inflammation of the mouth and gums as well as mouth eruptions.  In some cases, the genital area may be infected by Herpes simplex virus producing Herpes genitalis.  Herpes simplex virus may also infect the eye, producing acute keratitis which may permanently affect the cornea.

Following initial attack, symptoms disappear but the the virus survives in latent form in its host, probably in ganglia.  Recurrent attacks may follow nonspecific stimuli such as fever.  It is thought that a deficiency of the immune system of the host is the most significant factor in reactiviating the virus.

The virus is transmitted by contamination with droplets e.g., from saliva, or by direct contact.  It is estimated that 70-90% of all adults have antibodies against the virus. However, the antibodies appear to be ineffective in preventing recurrences.

Although it was once thought that only a single type of Herpes simplex virus existed, with possibly some minor antigenic variants, it is now known that there are two major types of the virus, differing antigenically and biologically. Herpes simplex virus Type I is an oral type and is usually the cause of Herpes labialis. Herpes simplex virus Type II is a genital type and is usually the cause of Herpes genitalis. However, Herpes simplex virus Type I has been isolated from genital lesions in some instances, while Herpes simplex virus Type II has been isolated from lesions on other parts of the body.

Herpes genitalis is a distressing condition. It is the second most common veneral disease in young adults. Herpes genitalis often causes severe psychological and social problems for affected individuals. Infection can be fatal in patients with deficient immune systems. Herpes genitalis has been associated with cancer of the genital organs in both men and women. It also poses the threat of infection to newborns of infected mothers.

At present, there is no known effective therapy against Herpes labialis and Herpes genitalis, although some antiherpetic agents, such as trifluorothymidine, idoxouridine, and cytosine arabinoside, have been reported to be effective in the treatment of acute keratitis caused by Herpes simplex infections of the eye. However, these drugs are toxic and some individuals are allergic to these drugs. Furthermore, some of these drugs, such as idoxouridine, are indicated only in the treatment of Herpes simplex keratitis. Studies report that treatment with these drugs suppresses but does not eradicate the virus and that relapse may occur when treatment ceases.

It would thus be desirable to provide a pharmaceutical agent which is effective against Herpes labialis and Herpes genitalis.

It would also be desirable if the pharmaceutical agent which is effective against Herpes labialis and Herpes genitalis is also nontoxic.

It would also be desirable if the pharmaceutical agent reduced the incidence of recurrent attacks of Herpes labialis and Herpes genitalis.

Interferons are proteins produced by cells in response to the action of specific inducers, such as viruses. They may be produced in vivo by living organisms or in vitro by tissue cultures in response to the presence of the inducer. There are three main varieties of human interferon: leukocyte (also known as $\alpha$), fibroblast (also known as $\beta$), and immune (also known as $\gamma$) interferon. There are now known to be several subvarieties of human leukocyte ($\alpha$) and fibroblast ($\beta$) interferon.

The interferons are known to be effective antiviral agents. Further, the interferons are known to be effective against a broad spectrum of viruses. They are also nontoxic and nonantigenic. Thus, the interferons offer hope as effective nontoxic pharmaceutical agents against herpetic lesions.

Up to now, treatment of herpetic lesions with interferon has proceeded along two main courses: a) medicinal induction of endogenic interferon in the patient; and b) administration of exogenic interferon to the patient.

For example, U.S. 4,053,582 (Stickl) discloses a method of treating Herpes genitalis and Herpes labialis by administering attenuated fowl pox virus to the patient. The attenuated fowl pox virus induces the patient to produce interferon. Within a few days, the eruptions caused

- 4 -

by Herpes simplex virus are healed.

U.S. 4,061,538 (Dorner et al.) and 4,184,917 (Dorner et al.) disclose a method of treating Herpes simplex viral infections in rabbits by systemic administration of a structurally modified interferons. These patents disclose that the modified interferons may be systemically administered to the host by injection or parenterally, for example, by intravenous administration.

Thus, none of these patents discloses a method of treating Herpes labialis or Herpes genitalis by topical administration of interferon to the lesions. Because viral multiplication occurs at the site of lesions, it is expected that local administration of human interferon to herpetic lesions is a more effective method of treating Herpes labialis and Herpes genitalis than previously disclosed methods.

Several published reports already disclose the treatment of herpetic eye infections by the topical administration of human interferon. For example, D. Neumann-Haefelin, et al., in Infection and Immunity, 17, 468 (1977), disclose that topically applied human leukocyte and fibroblast interferon prevents Herpes simplex Type I virus-induced keratitis in monkeys. B.R. Jones, et al., in Lancet ii, 128 (1976) report that topically applied purified human leukocyte interferon is effective for treating herpetic keratitis in humans. R. Sundmacher, et al., in Lancet ___, ( ), disclose that herpetic keratitis in humans can be treated by the topical administration of leukocyte interferon and triflurothymidine.

However, none of these publications discloses the treatment of Herpes labialis and Herpes genitalis by the topical administration of human interferon. Moreover, none of these publications discloses interferon-containing pharmaceutical preparations suitable for treating herpetic lesions.

Accordingly, it is an object of the present invention to provide a method for treating lesions due to Herpes labialis and Herpes genitalis by topically applying human interferon to the lesions.

It is also an object of the present invention to provide interferon-containing pharmaceutical preparations which are suitable for treating herpetic lesions by topically applying the preparations to the lesions.

How these and other objects of this invention are achieved will become apparent in light of the accompanying disclosure and claims.

## SUMMARY OF THE INVENTION

A method for treating <u>Herpes</u> <u>labialis</u> and <u>Herpes</u> <u>genitalis</u> lesions according to the present invention comprises administering locally an effective amount of a pharmaceutical preparation containing human interferon to the lesions. For purposes of the present invention, an effective amount of human interferon comprises about $1 \times 10^6 - 100 \times 10^6$, preferably $10 \times 10^6 - 50 \times 10^6$, I.U. per day.

Pharmaceutical preparations in accordance with the present invention comprise human interferon and a physiologically acceptable carrier, such as distilled water. Desirably, the pharmaceutical preparations further comprise an effective amount of a stabilizer, a preservative, an antibiotic, an anti-viral agent, or an anti-inflammatory agent.

## DETAILED DESCRIPTION OF THE INVENTION

In its latent form, Herpes simplex virus resides in the neurones of sensory ganglia. Upon reactivation, viral multiplication occurs at the site of the lesion. Thus, lesions due to Herpes labialis and Herpes genitalis may be treated rapidly and effectively by topically administering a human interferon-containing composition in an effective dosage to the blistered lesions.

In the practice of the present invention, comprising the treatment of Herpes labialis and Herpes genitalis lesions by topically administering human-interferon containing composition to the lesions, any of the known human interferons can be employed. Thus, the composition may contain human leukocyte, fibroblast, or immune interferon or mixtures thereof in suitable dosage. The interferon may suitably be prepared by well-known "classical" methods or by recombinant DNA methods.

Satisfactory results in healing the herpetic lesions are obtained when the human interferon is administered locally at a high concentration in a short period of time. An effective dosage for treating herpetic lesions is about $1 \times 10^6 - 100 \times 10^6$, perferably about $10 \times 10^6 - 50 \times 10^6$, I.U. per day. It may be perferable to apply the interferon-containing composition in a single daily dose rather than in several smaller doses. No special preparation or pretreatment is required prior to local administration of the human interferon. Generally the lesions are noticeably improved within hours of local administration of the human interferon. The lesions are completely healed within 48 hours and the incidence of recurrent attacks is significantly diminished.

Local administration of the interferon may be effected by directly applying an interferon-containing composition to or in areas adjacent to the Herpes labialis or Herpes genitalis lesions. Since it is generally desirable that the interferon be immediately released into the site of the lesions, it is preferable to apply the interferon in liquid form, for example by suspending it in an aqueous physiologically acceptable carrier. After a suitable period of time, the interferon-containing composition can readily be removed from the lesions, for example by washing. Distilled water is the preferred carrier. Other suitable carriers include a saline solution, a buffer solution, alcohol, etc. The interferon-containing composition may also be directly applied to the lesions in the form of an aerosol when combined in liquid form with a propellant.

Where slow release of the interferon is desired, the interferon may be applied as an ointment or cream to the lesions. For example, the interferon may be incorporated in an oil-in-water emulsion or in a physiologically acceptable hydrophilic carrier. When very slow release of the interferon is desired, it may be incorporated in an aqueous carrier and then formed into a water-in-oil emulsion or it may be incorporated in a physiologically acceptable hydrophobic carrier. Physiologically acceptable carriers include gelatine, lactose, starch, magnesium stearate, vegetable oils, benzyl alcohol, gums, polyalkylene glycols, white petroleum jelly, etc.

Local administration of the interferon may also be effected by subcutaneous injection directly into or in areas closely adjacent to the lesions. For injection, the interferon should be in liquid form, i.e. associated with an aqueous physiologically acceptable carrier such as distilled water.

In addition to the carrier, an interferon-containing pharmaceutical preparation according to the present invention may additionally contain suitable amounts of desirable adjuvants. For example, the preparation may desirably contain a stabilizer which increases the antiviral effectiveness of interferon. Bovine serum albumin (BSA) is a preferred stabilizer because it increases the effectiveness of interferon by adsorbing it to retain its conformation. The preparation may also desirably contain a preservative, such as glycerine.

It may further be desirable to incorporate additional therapeutic agents, for example, suitable antibiotics, in the interferon-containing pharmaceutical preparations. For example, a mixture of bacitracin and neomycin sulfate in effective dosages may suitably be included in the preparation. In this way, it is possible to protect the patient against infection by ordinary germs while being treated for Herpes labialis and Herpes genitalis.

It may further be desirable to include in suitable dosage a broad spectrum anti-viral agent in the interferon-containing composition. Acyclovir is a suitable anti-viral agent of this category. Alternatively, it may be desirable to include in suitable dosage a more specific antiherpetic agent, such as trifluorothymidine.

It may further be desirable to include in the pharmaceutical preparation a suitable dosage of an anti-inflammatory agent. Anti-inflammatory agents, such as aspirin, reduce the pain accompanying the lesions. Anti-inflammatory agents, such as the proteolytic enzyme bromelaine, are believed to speed healing and accelerate tissue repair, as well as ease pain. Other suitable anti-flammatory also may be included in the interferon-containing pharmaceutical preparation.

It should be emphasized that treatment of Herpes labialis and Herpes genitalis in accordance with the practices of the present invention does not eradicate Herpes simplex virus from its host. The virus continues to reside in latent form in the neurones of sensory ganglia. However, local administration of human interferon-containing compositions retards viral multiplication at the site of the lesions and promotes healing of the lesions. In addition, local administrations of human interferon-containing compositions appears to reduce recurrences of Herpes labialis and Herpes genitalis.

The following examples are intended to illustrate more clearly the practices of the present invention.

## Example 1

Patient A, a female suffering from recurrent attacks of Herpes genitalis, may be treated with human interferon-containing pharmaceutical preparations in accordance with the present invention. On the average, manifestations occur every 8 weeks and symptoms last for about 10 days. At the next outbreak, she is treated by directly applying a pharmaceutical preparation containing $1 \times 10^8$ I.U. human leukocyte interferon per ml of distilled water as a carrier. Three drops are applied at intervals of 6 hours. Within 4 hours there is noticeable improvement. Within 48 hours the eruptions are completely healed. Treatment continues for another 48 hours. The patient is free of recurrences and complaints for the next six months.

## Example 2

Patient B, a male suffering from recurrent attacks from Herpes labialis, may be treated in accordance with practices of the present invention. On the average, manifestations occur every 6 months and last for 10 days. The patient is treated once a day with three drops of the pharmaceutical preparation of Example 1. The skin eruptions start to dry up within hours and are completely gone within 48 hours.

## Example 3

Patient C, a male suffering from recurrent attacks of Herpes genitalis, may be treated in accordance with the practices of the present invention. On the average, manifestations recur frequently. The patient is treated with a pharmaceutical preparation containing human leukocyte interferon of high activity ($1x10^8$ I.U./mg.) and the antiviral agent acyclovir in a Neobase carrier. The ointment is applied at 6 hour intervals. Noticeable improvement occurs within hours and the lesions are completely healed within 48 hours.

While the invention has been described by reference to specific embodiments, this was for purposes of illustration only and should not be construed to limit the spirit or scope of the invention.

-12-

WHAT IS CLAIMED IS:

1.    A pharmaceutical preparation for the treatment of
_Herpes_ _labialis_ or _Herpes_ _genitalis_ lesions comprising an
effective amount of human interferon and a physiologically
acceptable carrier.

2.    The pharmaceutical preparation of Claim 1 wherein
said human interferon comprises human leukocyte, fibroblast, or immune interferon.

3.    The pharmaceutical preparation of Claim 1 wherein
said carrier comprises an aqueous physiologically acceptable substance.

4.    The parmaceutical preparation of Claim 3 wherein
said carrier comprises distilled water, a buffer solution,
or a saline solution.

5.    The pharmaceutical preparation of Claim 1 wherein
said carrier comprises a hydrophilic substance.

6.    The pharmaceutical preparation of Claim 1 wherein
said carrier comprises a hydrophobic substance.

7.    The pharmaceutical preparation of Claim 1 further
comprising a stabilizer.

8.    The pharmaceutical preparation of Claim 7 wherein
said stabilizer is bovine serum albumin.

9.    The pharmaceutical preparation of Claim 1 further
comprising a preservative.

10.   The pharmaceutical preparation of Claim 9 wherein
said preservative is glycerine.

11. The pharmaceutical preparation of Claim 1 further comprising an effective amount of an antibiotic.

12. The pharmaceutical preparation of Claim 11 wherein said antibiotic comprises bacitracin or neomycin sulfate.

13. The pharmaceutical preparation of Claim 1 further comprising an effective amount of an additional anti-viral agent.

14. The pharmaceutical preparation of Claim 13 wherein said additional anti-viral agent is acyclovir.

15. The pharmaceutical preparation of Claim 1 further comprising an effective amount of an anti-flammatory agent.

16. The pharmaceutical preparation of Claim 15 wherein said anti-inflammatory agent is aspirin or a proteolytic enzyme.

17. A pharmaceutical preparation for treating herpetic lesions comprising human interferon, a physiologically acceptable carrier therefor, a stabilizer for said interferon and an anti-inflammatory agent, said human interferon being present in said composition in an effective anti-viral amount.

18. The preparation in accordance with Claim 17 wherein said composition also includes an anti-viral agent in addition to human interferon.

19. The preparation in accordance with Claim 17 wherein said anti-inflammatory agent is a prostaglandin biosynthesis inhibitor.

20.   The preparation in accordance with Claim 17 wherein said anti-inflammatory agent is a steroid.

21.   The preparation in accordance with Claim 20 wherein said steriod is a prednisone or a prednisolone.